# EUROPÄISCHE PATENTSCHRIFT

(11) **EP 3 634 533 B1**
(45) Veröffentlichungstag und Bekanntmachung des Hinweises auf die Patenterteilung: **15.11.2023**
(21) Anmeldenummer: 18731026.3
(22) Anmeldetag: 07.06.2018
(51) Int. Cl.: A61M 1/36

(54) **VORRICHTUNG ZUR EXTRAKORPORALEN BLUTBEHANDLUNG UND ZUM BESTIMMEN EINES HÄMODYNAMISCHEN PARAMETERS WÄHREND EINER EXTRAKORPORALEN BLUTBEHANDLUNG**
DEVICE FOR AN EXTRACORPOREAL BLOOD TREATMENT AND FOR DETERMINING A HEMODYNAMIC PARAMETER DURING AN EXTRACORPOREAL BLOOD TREATMENT
DISPOSITIF POUR LE TRAITEMENT EXTRACORPOREL DU SANG ET POUR DÉTERMINER UN PARAMÈTRE HÉMODYNAMIQUE PENDANT UN TRAITEMENT EXTRACORPOREL DU SANG

(30) Priorität: 10.06.2017 DE 102017005534
(43) Veröffentlichungstag der Anmeldung: 15.04.2020
(73) Patentinhaber: Fresenius Medical Care Deutschland GmbH, 61352 Bad Homburg v.d.H (DE)
(72) Erfinder: MAIERHOFER, Dr. Andreas, 97422 Schweinfurt (DE)
(74) Vertreter: Oppermann, Frank
(86) Internationale Anmeldenummer: PCT/EP2018/065066
(87) Internationale Veröffentlichungsnummer: WO 2018/224606

(56) Entgegenhaltungen:
- WO-A1-2018/001996
- DE-C1- 19 528 907

## Beschreibung

Die Erfindung betrifft eine Vorrichtung zur extrakorporalen Blutbehandlung nach Anspruch 1.

Bei Verfahren der chronischen Blutreinigungstherapie, wie Hämodialyse, Hämofiltration und Hämodiafiltration, wird Blut über einen extrakorporalen Blutkreislauf geleitet. Bei der Hamödialyse durchströmt das zu behandelnde Blut die Blutkammer eines durch eine semipermeable Membran in die Blutkammer und eine Dialysierflüssigkeitskammer unterteilten Dialysators, während in einem Dialysierflüssigkeitssystem Dialysierflüssigkeit die Dialysierflüssigkeitskammer des Dialysators durchströmt. Der extrakorporale Blutkreislauf weist eine arterielle Schlauchleitung auf, die zu der Blutkammer führt, und eine venöse Schlauchleitung auf, die von der Blutkammer abgeht. Die bekannten Blutbehandlungsvorrichtungen verfügen über eine Blutpumpe, die in der Regel stromauf der Blutkammer des Dialysators angeordnet ist, um einen ausreichenden Blutfluss im extrakorporalen Blutkreislauf sicherzustellen.

Als Zugang zum Blutgefäßsystem des Patienten wird häufig operativ eine arteriovenöse Fistel angelegt. Ebenso ist der Einsatz eines Implantats möglich. Für die Funktionsfähigkeit der Fistel ist deren Perfusion von Bedeutung. Sinkt der Fistelfluss unter einen kritischen Wert, dann steigt das Risiko einer Fistelthrombose mit dem möglichen Verlust des Gefäßzuganges, was in der Dialysebehandlung eine erhebliche Komplikation darstellt (W. Bay et al.: Color Doppler flow predicts PTFE graft failure, J. Am. Soc. Nephrol. 5: 407 (1994)). Ist der Fistelfluss während der Dialysebehandlung kleiner als der extrakorporale Blutfluss (QB), kommt es zur lokalen Fistelrezirkulation, wobei eine Fraktion des dialysierten und mit der venösen Blutleitung zur Fistel zurückgeführten Bluts über die arterielle Blutleitung dem Dialysator wieder zugeführt wird. Die Fistelrezirkulation (RA) bewirkt eine bedeutende Verminderung der Dialyseeffizienz (F. Gotch: "Models to predict recirculation and its effect on treatment time in single-needle dialysis" First Intl. Symposium on Single-Needle Dialysis, Hrsg.: S. Rignoir. R. Vanholder und P. Invanovich, Cleveland, ISAO Press, 1984, Seite 305 ff.).

Aufgrund ihrer klinischen Bedeutung sind verschiedene Verfahren zur Bestimmung des Blutflusses im Gefäßzugang (Shuntfluss) oder der Rezirkulation bekannt. Viele Verfahren basieren auf der Messung einer physikalischen oder chemischen Kenngröße des Bluts, die im extrakorporalen Blutkreislauf verändert wird. Die physikalische oder chemische Kenngröße kann beispielsweise die Temperatur, Dichte oder Elektrolytzusammensetzung des Bluts sein. Die Kenngröße kann im extrakorporalen Blutkreislauf unmittelbar oder mittelbar durch eine Änderung einer physikalischen oder chemischen Kenngröße im Dialysierflüssigkeitssystem verändert werden.

Aus EDTNA-ERCA Journal 19, 6 (1993) ist ein als Thermodilution bezeichnetes Verfahren zur Shuntflussmessung bekannt. Bei dem bekannten Verfahren wird ein kurzzeitiger Temperaturabfall im Dialysierflüssigkeitssystem initiiert, der sich auf den venösen Zweig des extrakorporalen Kreislaufs überträgt und zu einem nachweisbaren Temperatursprung im arteriellen Zweig des extrakorporalen Kreislaufs dann führt, wenn eine Rezirkulation auftritt.

Die DE 195 28 907 C1 beschreibt ein Verfahren zum Bestimmen der kardiopulmonalen Rezirkulation, das auf zwei aufeinanderfolgenden Messungen der Rezirkulationsfraktion beruht, die vor und nach der Umkehr des Blutflusses im extrakorporalen Kreislauf durchgeführt werden. Eine Umkehrung des Blutflusses kann grundsätzlich durch Vertauschen der arteriellen und venösen Patientenanschlüsse erfolgen. Das manuelle Vertauschen der Patientenanschlüsse ist aber unter hygienischen und sicherheitstechnischen Gesichtspunkten nicht unproblematisch. Daher sind zahlreiche Vorrichtungen für Blutschlauchsysteme entwickelt worden, die eine automatische Umkehr der Flussrichtung durch eine kreuzweise Verschaltung der betreffenden Schlauchleitungsabschnitte des Schlauchleitungssystems bewirken. Dabei wird aber die Flussrichtung in dem Teil des extrakorporalen Kreislaufs, der den Dialysator einschließt, beibehalten. Daher können die in diesem Teil befindlichen Schutzsysteme sowohl vor als auch nach der Umkehr des Blutflusses verwendet werden.

Eine Ventileinrichtung für die automatische Umkehr der Flussrichtung ist beispielsweise aus der WO 2014/074231 A1 bekannt.

Die DE 195 28 907 C1 schlägt als eine Alternative zu den bekannten Ventileinrichtungen für die automatische Umkehr der Flussrichtung vor, einfach die Förderrichtung der Blutpumpe im extrakorporalen Blutkreislauf umzukehren. Allerdings ist von dieser Möglichkeit in der Praxis bisher kein Gebrauch gemacht worden, da sich der Betrieb einer extrakorporalen Blutbehandlungsvorrichtung mit einem Dialysator, der von Blut in unterschiedlichen Richtungen durchströmt wird, unter sicherheitstechnischen Gesichtspunkten als nicht unproblematisch erweist.

Die WO2018/001996 beschreibt eine Vorrichtung zur extrakorporalen Blutbehandlung, wobei eine Blutpumpe für einen Messzyklus in einem Betriebsmodus mit einem umgekehrten Blutfluss betrieben wird.

Der Erfindung liegt die Aufgabe zugrunde, eine Messung eines hämodynamischen Parameters ohne die Verwendung der bekannten Ventileinrichtungen für die automatische Umkehr der Flussrichtung zu ermöglichen, die eine automatische Umkehr der Flussrichtung durch eine kreuzweise Verschaltung der betreffenden Schlauchleitungsabschnitte des Schlauchleitungssystems bewirken.

Die Lösung dieser Aufgabe erfolgt mit den Merkmalen der unabhängigen Patentansprüche. Die abhängigen Ansprüche betreffen bevorzugte Ausführungsformen der Erfindung.

Zur Vermeidung der Bildung von Blutgerinnseln im extrakorporalen Blutkreislauf wird dem Blut des Patienten häufig eine gerinnungshemmende Substanz, meist Heparin, entweder kontinuierlich während der Blutbehandlung oder zu Beginn der Blutbehandlung als initialer Bolus zugegeben. Die Zugabe erfolgt üblicherweise stromauf des Dialysators. Neben der systemischen Wirkung der gerinnungshemmenden Substanz im Patienten ist ein möglicher Wirkmechanismus auch die Anlagerung dieser Substanz an die Oberflächen des extrakorporalen Systems, insbesondere an das Lumen der Dialysatorfasern.

Der bei einer extrakorporalen Blutbehandlung verwendete Dialysator stellt wegen des Durchmessers der Hohlfasern von kleiner als 0,2 mm ein für Gerinnsel unüberwindliches Hindernis dar. Daher können stromauf des Dialysators sich bildende Gerinnsel nicht in den Bereich stromab des Dialysators und somit nicht in den Patienten gelangen. Allerdings können stromab des Dialysators befindliche Blutgerinnsel infundiert werden. Dies ist unabhängig von der Flussrichtung des Bluts durch den Dialysator.

Bei den bekannten Blutbehandlungsvorrichtungen werden sich im Blut auf dem Weg vom Dialysator zum Patienten bildende Gerinnsel im Allgemeinen mit einer Vorrichtung zum Fangen von Blutgerinnseln zurückgehalten, die stromab des Dialysators in der venösen Blutleitung des extrakorporalen Schlauchsystems vor der venösen Kanüle vorgesehen ist. Diese Vorrichtung wird auch als Blutgerinnselfänger bezeichnet.

In der Praxis hat sich gezeigt, dass bei einer Umkehr der Förderrichtung der Blutpumpe für eine Messung zur Bestimmung eines hämodynamischen Parameters das Risiko besteht, dass Blutgerinnsel in den Patienten gelangen, obwohl der Dialysator sich stromauf des Dialysators bildende Blutgerinnsel zurückhält. Es hat sich in der Praxis gezeigt, dass sich während eines Betriebs mit einem "normalen" Blutfluss am Eingang des Dialysators Blutgerinnsel ansammeln. Bei einer Umkehr des Blutflusses werden diese Blutgerinnsel dann in Richtung des Patienten gespült.

Für die Erfindung ist unerheblich, welche hämodynamischen Parameter auf der Grundlage von einer oder mehreren Messungen bei einem umgekehrten Blutfluss bestimmt werden und wie die hämodynamischen Parameter bestimmt werden. Unerheblich ist auch, ob blutseitige oder dialysatseitige Größen gemessen werden. Anstelle der physikalischen oder chemischen Eigenschaft des Bluts in der Blutleitung kann auch eine mit der physikalischen oder chemischen Kenngröße in der Blutleitung korrelierende Kenngröße im Dialysat gemessen werden. Die Erfindung bezieht sich insofern auf sämtliche Verfahren, bei denen vor und nach der Umkehr der Flussrichtung oder nur bei einem umgekehrten Blutfluss eine chemische oder physikalische Kenngröße gemessen wird, um den hämodynamischen Parameter zu bestimmen.

Die Erfindung bezieht sich beispielsweise auf sämtliche Verfahren, bei denen vor und nach der Umkehr der Flussrichtung eine chemische oder physikalische Kenngröße im Blut in einem Zweig des extrakorporalen Blutkreislaufs verändert und die auf die Veränderung der chemischen oder physikalischen Kenngröße in diesem Zweig zurückzuführende Änderung der chemischen oder physikalischen Kenngröße im Blut in einem anderen Zweig des extrakorporalen Blutkreislaufs oder im Dialysat erfasst wird.

Für die Bestimmung des hämodynamischen Parameters kann der Verlauf der beobachtbaren zeitlichen Veränderung der physikalischen oder chemischen Kenngröße im blutseitigen oder dialysatseitigen Leitungssystem gemessen werden. Ein konkreter Anwendungsfall ist die Bestimmung der Rezirkulation nach den bekannten Verfahren, die eine Umkehr der Flussrichtung voraussetzen. Der hämodynamische Parameter kann dabei die Rezirkulation in der Fistel oder der Shuntfluss sein.

Bei der Blutbehandlungseinheit kann es sich um eine beliebige Einheit zur Durchführung einer Hämodialyse, Hämofiltration, Hämodiafiltration, Apherese o.ä., handeln. Im Fall einer Hämodialyse, Hämofiltration, Hämodiafiltration weist die Blutbehandlungseinheit (Dialysator) ein erstes und ein zweites Kompartiment auf, die durch eine semipermeable Membran voneinander getrennt sind. Wenn die Blutbehandlung hingegen nicht auf der einem diffusiven oder konvektiven Stoffaustausch, beispielsweise auf einer Adsorption an funktionalisierten Oberflächen beruht, so ist ein zweites Kompartiment nicht erforderlich.

Die erfindungsgemäße Vorrichtung zur extrakorporalen Blutbehandlung umfasst:
eine Blutbehandlungseinheit, die mindestens ein Kompartiment aufweist,
eine an einen Anschluss des Kompartiments angeschlossenen erste Blutleitung, die einen ersten Patientenanschluss aufweist,
eine an einen Anschluss des Kompartiments angeschlossene zweite Blutleitung, die einen zweiten Patientenanschluss aufweist,
eine Blutpumpe zum Fördern von Blut,
eine Einrichtung zum Messen einer physikalischen oder chemischen Kenngröße, und
eine mit der Blutpumpe und der Einrichtung zum Messen einer physikalischen oder chemischen Kenngröße verbundenen Steuer- und Auswerteeinheit.

Eine erste Ausführungsform der Blutbehandlungsvorrichtung sieht eine Bestimmung des hämodynamischen Parameters auf der Grundlage einer Messung mit zwei Messzyklen vor. Bei dieser Ausführungsform ist die Steuer- und Auswerteeinheit derart konfiguriert, dass die Blutpumpe für einen Messzyklus von zwei Messzyklen in einem Betriebsmodus mit einem umgekehrten Blutfluss betrieben wird, so dass Blut von dem zweiten Patientenanschluss zu der Blutbehandlungseinheit und von der Blutbehandlungseinheit zu dem ersten Patientenanschluss strömt, und die Blutpumpe für den anderen Messzyklus der beiden Messzyklen in einem Betriebsmodus mit einem normalen Blutfluss betrieben wird, so dass Blut von dem ersten Patientenanschluss zu der Blutbehandlungseinheit und von der Blutbehandlungseinheit zu dem zweiten Patientenanschluss strömt, wobei aus der gemessenen physikalischen oder chemischen Kenngröße in dem Messzyklus bei normalem und umgekehrtem Blutfluss der hämodynamische Parameter bestimmt wird. Der Messzyklus mit normalem Blutfluss kann dem Messzyklus mit umgekehrtem Blutfluss vorausgehen oder der Messzyklus mit umgekehrtem Blutfluss kann dem Messzyklus mit normalem Blutfluss vorausgehen.

Eine alternative Ausführungsform der Blutbehandlungsvorrichtung sieht eine Bestimmung des hämodynamischen Parameters auf der Grundlage nur einer Messung in einem Betriebsmodus mit einem umgekehrten Blutfluss vor, so dass Blut von dem zweiten Patientenanschluss zu der Blutbehandlungseinheit und von der Blutbehandlungseinheit zu dem ersten Patientenanschluss strömt, wobei aus der gemessenen physikalischen oder chemischen Kenngröße in dem Messzyklus bei umgekehrtem Blutfluss der hämodynamische

### Parameter bestimmt wird.

Beiden Messmethoden ist aber gemeinsam, dass die Blutpumpe in einem Betriebsmodus mit einem umgekehrten Blutfluss betrieben wird, in dem grundsätzlich die Gefahr der Gerinnselbildung besteht, da bei den bekannten Blutbehandlungsvorrichtungen eine Vorrichtung zum Sammeln von Gerinnseln in der von dem Dialysator zu dem zweiten Patientenanschluss führenden Blutleitung im Allgemeinen nicht vorhanden ist.

Die erfindungsgemäße Vorrichtung beruht darauf, für die Bestimmung des hämodynamischen Parameters eine Umkehrung des Blutflusses vorzunehmen, obwohl grundsätzlich die Gefahr einer Bildung von Blutgerinnseln besteht. Zu Beginn der Blutbehandlung ist der extrakorporale Blutkreislauf frei von Blutgerinnseln. In der Praxis hat sich gezeigt, dass Blutgerinnsel erst in einem relativ späten Verlauf der Blutbehandlung auftreten. Die Zeitspanne, in der sich Blutgerinnsel noch nicht gebildet haben, ist von verschiedenen patientenspezifischen und systemspezifischen Faktoren abhängig. Hierzu gehören die allgemeine Gerinnungsneigung des Blutes des Patienten, Art und Menge der eingesetzten gerinnungshemmenden Substanzen (Antikoagulanzien), die Oberflächenbeschaffenheit der Teile, mit denen das Blut im extrakorporalen Blutkreislauf in Kontakt kommt, sowie die Intensität des Luftkontaktes des Blutes im extrakorporalen Blutkreislauf.

Die erfindungsgemäße extrakorporale Blutbehandlungsvorrichtung weist eine Eingabeeinheit zur Eingabe eines Zeitintervalls auf, das der Anwender unter Berücksichtigung der patientenspezifischen und systemspezifischen Faktoren vorgegeben kann. Die Steuer- und Auswerteeinheit der extrakorporalen Blutbehandlungsvorrichtung ist derart konfiguriert, dass der Betrieb der Blutpumpe in dem Betriebsmodus mit einem umgekehrten Blutfluss nur innerhalb des mit der Eingabeeinheit eingegebenen Zeitintervalls freigegeben ist.

Der Anfang des Zeitintervalls wird vorzugsweise von dem Zeitpunkt des Beginns der Blutbehandlung bestimmt. Der Beginn der Blutbehandlung kann als der Zeitpunkt festgelegt werden, zu dem der Blutfluss im extrakorporalen Blutkreislauf gestartet wird, beispielsweise wenn die Blutpumpe in Betrieb gesetzt wird und die venöse und arterielle Schlauchklemme geöffnet werden. Der Blutbehandlung kann eine Reinigungs- und/oder Testphase vorausgehen, um die Blutbehandlungsvorrichtung für die Blutbehandlung vorzubereiten. In dieser Phase kann der extrakorporale Blutkreislauf beispielsweise mit einer Spülflüssigkeit gespült werden.

Die Steuer- und Auswerteeinheit ist vorzugsweise derart konfiguriert, dass mit der Erzeugung eines Startsignals für die Blutbehandlung der Beginn des Zeitintervalls festgelegt wird. Beispielsweise kann die Steuer- und Auswerteeinheit derart konfiguriert, sein, dass das Startsignal mit dem Start der Blutpumpe und/oder dem Öffnen der venösen und arteriellen Schlauchklemme erzeugt wird. Zu diesem Zeitpunkt kann ein Zeitglied zu laufen beginnen, das den Betrieb der Blutpumpe in dem Betriebsmodus mit einem umgekehrten Blutfluss nur innerhalb des eingegebenen Zeitintervalls freigibt. Dieses Zeitglied kann Bestandteil der Steuer- und Auswerteeinheit sein.

Nach Ablauf des Zeitintervalls kann ein Betrieb der Blutpumpe mit einem umgekehrten Blutfluss nicht mehr erfolgen, so dass eine Gefährdung des Patienten nicht gegeben ist. Eine Bestimmung eines hämodynamischen Parameters zu einem späteren Zeitpunkt der Blutbehandlung, bei dem die Gefahr einer Gerinnselbildung besteht, ist daher ausgeschlossen. Der zu bestimmende hämodynamische Parameter kann beispielsweise die Rezirkulation oder der Shuntfluss sein.

Eine Ausführungsform sieht vor, dass die Steuer- und Auswerteeinheit derart konfiguriert ist, dass zu Beginn der Blutbehandlung die Blutpumpe in einem Betriebsmodus mit einem umgekehrten Blutfluss betrieben wird, wobei der hämodynamische Parameter aus der gemessenen physikalischen oder chemischen Kenngröße in dem Messzyklus bei umgekehrtem Blutfluss bestimmt wird. Die Blutbehandlung wird also mit einem umgekehrten Blutfluss begonnen. Eine weitere Ausführungsform sieht eine Umkehr des Blutflusses erst nach Ablauf einer initialen Phase der Blutbehandlung vor, in der die Blutpumpe mit einem normalen Blutfluss betrieben wird. In dieser Phase kann ein initialer Bolus einer gerinnungshemmenden Substanz, beispielsweise Heparin,
dem Blut zugegeben werden. Folglich kann die Blutbehandlung mit dem umgekehrten Blutfluss erst nach Verabreichen der gerinnungshemmenden Substanz beginnen.

Für die Zugabe einer gerinnungshemmenden Substanz in das Blut kann die Vorrichtung zur extrakorporalen Blutbehandlung eine Dosiereinrichtung aufweisen, wobei die Steuer- und Auswerteeinheit derart konfiguriert ist, dass die Dosiereinrichtung für die Zugabe einer gerinnungshemmenden Substanz in der initialen Phase der Blutbehandlung betrieben wird. Die Blutpumpe wird in der initialen Phase der Blutbehandlung vorzugsweise in einem Betriebsmodus mit einem normalen Blutfluss betrieben. Es ist aber auch möglich, die gerinnungshemmende Substanz mit der Dosiereinrichtung während der Blutbehandlung kontinuierlich zuzugeben. Dann kann die Blutbehandlung sofort mit umgekehrtem Blutfluss begonnen werden.

Die für eine Shuntflussmessung notwendige Zeitdauer ist stark von der verwendeten Messmethode abhängig. Bei den bekannten Verfahren zur Bestimmung eines hämodynamischen Parameters kann die Messdauer relativ kurz sein, wenn die physikalische oder chemische Kenngröße beispielsweise durch Zugabe eines Bolus einer bestimmten Substanz direkt im extrakorporalen Blutkreislauf verändert wird. In diesem Fall kann für eine genaue Bestimmung des Shuntflusses nach einer Messung mit einem umgekehrten Blutfluss eine weitere Messung mit einem normalen Blutfluss nicht mehr erforderlich sein, da innerhalb der relativ kurzen Messdauer der kardiopulmonare Anteil an der Rezirkulation zu vernachlässigen ist. In der Praxis kann die Shuntflussmessung unter 20 bis 30 Sekunden liegen. Es sind aber auch Verfahren zur Bestimmung des Shuntflusses bekannt, die eine relativ lange Messdauer erfordern. Dies ist beispielsweise bei den als Thermodilution bekannten Verfahren der Fall (EDTNA-ERCA Journal 19, 6 (1993). In der Praxis kann die Shuntflussmessung bei diesen Verfahren zwischen 6 und 10 Minuten dauern. In diesem Fall kann der Shuntfluss nach nur einer Messung mit einem umgekehrten Blutfluss aber zumindest abgeschätzt werden.

Bei einer alternativen Ausführungsform ist die Steuer- und Auswerteeinheit derart konfiguriert, dass zu Beginn der Blutbehandlung oder nach Ablauf einer initialen Phase der Blutbehandlung, in der die Blutpumpe mit einem normalen Blutfluss betrieben wird, die Blutpumpe für einen ersten Messzyklus in einem Betriebsmodus mit einem umgekehrten Blutfluss betrieben wird, und die Blutpumpe für einen zweiten Messzyklus in einem Betriebsmodus mit einem normalen Blutfluss betrieben wird, wobei der hämodynamische Parameter aus der gemessenen physikalischen oder chemischen Kenngröße in dem Messzyklus bei normalem und umgekehrtem Blutfluss bestimmt wird. Die alternative Ausführungsform erlaubt mit den bekannten Verfahren eine exakte Bestimmung des Shuntflusses unter Berücksichtigung der kardiopulmonaren Rezirkulation.

Die Steuer- und Auswerteeinheit kann derart konfiguriert sein, dass am Ende des Zeitintervalls ein Steuersignal erzeugt wird, wenn die Blutpumpe in dem Betriebsmodus mit einem umgekehrten Blutfluss betrieben wird bzw. betrieben werden soll. Dieses Steuersignal kann ein Signal sein, dass einen Eingriff in die Steuerung vornimmt, dass den Blutfluss in der umgekehrten Flussrichtung unterbricht oder eine Umkehr der Flussrichtung verhindert. Das Steuersignal kann auch eine Alarmeinheit ansteuern, die einen Alarm auslöst. Der Ablauf des Zeitintervalls kann auch auf einer Anzeigeeinheit angezeigt werden.

Die Steuer- und Auswerteeinheit kann einen Speicher aufweisen, wobei die Steuer- und Auswerteeinheit derart konfiguriert ist, dass das Zeitintervall aus der Eingabeeinheit in den Speicher eingelesen wird.

Die Eingabeeinheit kann eine Tastatur und/oder einen berührungsempfindlichen Bildschirm und/oder eine Vorrichtung zum Lesen eines Datenträgers aufweisen, auf dem für mehrere Patienten unterschiedliche Werte gespeichert sein können, die von dem Benutzer mit der Tastatur oder dem berührungsempfindlichen Bildschirm ausgewählt werden können. Die Eingabeeinheit kann auch Bestandteil einer Datenverarbeitungseinheit sein, die mit der Steuer- und Recheneinheit über ein Netzwerk verbunden sein kann.

Für den Betrieb bei einem normalen Blutfluss weist die Vorrichtung zur extrakorporalen Blutbehandlung vorzugsweise eine Vorrichtung zum Sammeln von Blutgerinnseln in der zweiten Blutleitung auf, so dass sich stromab des Dialysators bildende Blutgerinnsel nicht in den Patienten gelangen können. Die sich stromauf des Dialysators bildenden Blutgerinnsel werden bei normalen Blutfluss im Dialysator infolge des kleinen Lumens der Hohlfasern zurückgehalten.

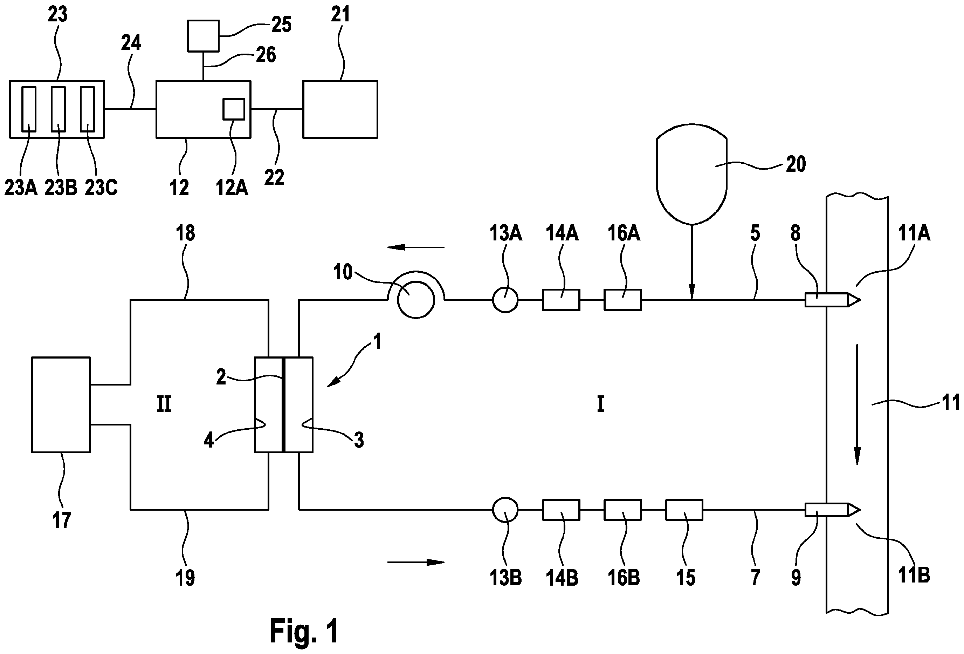

Im Folgenden wird die Erfindung unter Bezugnahme auf die einzige Figur näher erläutert, die eine Vorrichtung zur extrakorporalen Blutbehandlung in stark vereinfachter schematischer Darstellung zeigt.

Die Figur zeigt in stark vereinfachter schematischer Darstellung die für die Erfindung wesentlichen Komponenten der Blutbehandlungsvorrichtung, die bei dem vorliegenden Ausführungsbeispiel eine Hämodialysevorrichtung ist. Die Blutbehandlungsvorrichtung weist eine Blutbehandlungseinheit 1 auf, die ein Dialysator sein kann, der durch eine semipermeable Membran 2 in ein erstes Kompartiment 3 (Blutkammer) und ein zweites Kompartiment 4 (Dialysatkammer) unterteilt ist. Der extrakorporale Blutkreislauf I umfasst eine erste Blutleitung 5, die an einen ersten Anschluss des ersten Kompartiments 3 angeschlossen ist, das erste Kompartiment 3 und eine zweite Blutleitung 7, die an einen zweiten Anschluss des ersten Kompartiments 3 angeschlossen. Die erste und zweite Blutleitung 5, 7 sind Schlauchleitungen eines zur einmaligen Verwendung bestimmten Blutschlauchsystems (Disposable). An dem Ende der ersten Blutleitung 5 befindet sich ein erster Patientenanschluss 8 (Punktionskanüle) und an dem Ende der zweiten Blutleitung 7 befindet sich ein zweiter Patientenanschluss 9 (Punktionskanüle). Das Blut wird mit einer Blutpumpe 10 gefördert, die in der ersten Blutleitung 5 vorgesehen ist.

Zur Durchführung der Blutbehandlung wird die erste Punktionskanüle 8 an einem stromaufwärtigen Teil 11A des Gefäßzugangs 11 angeschlossen, während die zweite Punktionskanüle 9 an einem stromabwärtigen Teil 11B des Gefäßzugangs 11 angeschlossen wird, so dass das Blut des Patienten durch die erste Blutleitung 5, das erste Kompartiment 3 der Blutbehandlungseinheit 3 und die zweite Blutleitung 7 strömt. Dieser Fall wird als "normaler" Blutfluss bezeichnet. Die Blutpumpe 10 fördert dann in der "normalen" Förderrichtung. Der stromaufwärtige Teil 11A des Gefäßzugangs 11 stellt somit den arteriellen Teil der Fistel, die erste Blutleitung 5 die arterielle Blutleitung, die zweite Blutleitung 7 die venöse Blutleitung und der stromabwärtige Teil 11B der Fistel den venösen Teil der Fistel dar.

Für die Bestimmung des hämodynamischen Parameters, insbesondere des Shuntflusses, ist eine Umkehr der Flussrichtung erforderlich, so dass das Blut von dem stromabwärtigen Teil 11B des Gefäßzugangs 11 durch die zweite Blutleitung 7, das erste Kompartiment 3 der Blutbehandlungseinheit 1, die erste Blutleitung 5 zu dem stromaufwärtigen Teil 11A des Gefäßzugangs 11 strömt. Dieser Fall wird als "umgekehrter" Blutfluss bezeichnet. Die Blutpumpe 10 fördert dann in der umgekehrten Förderrichtung.

Die Blutbehandlungsvorrichtung verfügt über eine zentrale Steuer- und Auswerteeinheit 12 zur Steuerung sämtlicher Komponenten der Vorrichtung. Die Steuer- und Auswerteeinheit 12 kann beispielsweise einen allgemeinen Prozessor, einen digitalen Signalprozessor (DSP) zur kontinuierlichen Bearbeitung digitaler Signale, einen Mikroprozessor, eine anwendungsspezifische integrierte Schaltung (ASIC), einen aus Logikelementen bestehenden integrierten Schaltkreis (FPGA) oder andere integrierte Schaltkreise (IC) oder Hardware-Komponenten aufweisen, um die einzelnen Verfahrensschritte zur Steuerung der Blutbehandlungsvorrichtung auszuführen. Auf den Hardware-Komponenten kann zur Durchführung der Verfahrensschritte ein Datenverarbeitungsprogramm (Software) laufen. Es ist auch eine Mehrzahl oder Kombination der verschiedenen Komponenten möglich.

Die Steuer- und Auswerteeinheit 12 ist derart konfiguriert, dass die einzelnen Komponenten der Blutbehandlungsvorrichtung für die Durchführung der Blutbehandlung angesteuert und die Messungen für die Bestimmung eines hämodynamischen Parameters der Blutbehandlung durchgeführt werden.

Die Blutbehandlungsvorrichtung weist in der ersten Blutleitung 5 zwischen der Blutpumpe 10 und dem ersten Patientenanschluss 8 einen ersten Drucksensor 13A zum Messen des Drucks und in der zweiten Blutleitung 7 zwischen dem ersten Kompartiment 3 und dem zweiten Patientenanschluss 9 einen zweiten Drucksensor 13B zum Messen des Drucks auf.

Die Messungen für die Bestimmung eines hämodynamischen Parameters der Blutbehandlung können im extrakorporalen Blutkreislauf I durchgeführt werden. Zum Messen einer physikalischen oder chemischen Eigenschaft des Bluts in der ersten Blutleitung 5 ist eine erste Messeinrichtung 14A und zum Messen einer physikalischen oder chemischen Eigenschaft des Bluts in der zweiten Blutleitung 7 ist eine zweite Messeinrichtung 14B vorgesehen. Die Drucksensoren 13A, 13B und die Einrichtungen 14A, 14B zum Messen einer physikalischen oder chemischen Eigenschaft des Bluts sind über nicht dargestellte Datenleitungen mit der Steuer- und Auswerteeinheit 12 verbunden. In der zweiten Blutleitung 7 ist zwischen der zweiten Einrichtung 14B zum Messen einer physikalischen oder chemischen Eigenschaft und dem zweiten Patientenanschluss 9 eine Vorrichtung 15 zum Fangen von Blutgerinnseln vorgesehen. Der Blutgerinnselfänger 15 kann aber auch in einem anderen Abschnitt der zweiten Blutleitung 7 vorgesehen sein.

Die Blutbehandlungsvorrichtung kann weitere nur andeutungsweise dargestellte Überwachungs- und Sicherheitseinrichtungen 16A, 16B aufweisen, die sowohl in der "normalen" als auch "umgekehrten" Flussrichtung funktionsfähig sind. Zu diesen Überwachungs- und Sicherheitseinrichtungen 16A, 16B zählen eine Luftabscheidekammer, ein Luftdetektor und ein Absperrorgan in der ersten und zweiten Blutleitung. Die Luftabscheidekammern können Ein- und Auslassleitungen haben, die unterhalb des Flüssigkeitsspiegels angeordnet sind. Zur Erkennung von Luft können Ultraschallsensoren verwendet werden.

Darüber hinaus weist die Blutbehandlungsvorrichtung eine nur schematisch dargestellte Dosiereinrichtung 20 für die Zugabe einer gerinnungshemmenden Substanz (Antikoagulanz), beispielsweise Heparin, in das Blut des Patienten auf. Die Zugabe der gerinnungshemmenden Substanz erfolgt bei einem normalen Blutfluss in der ersten Blutleitung 5 stromauf der Blutbehandlungseinheit 1. Die Dosiereinrichtung 20 kann beispielsweise eine Spritzenpumpe sein, die über eine Schlauchleitung mit der ersten Blutleitung verbunden ist. In die Spritzenpumpe kann eine mit Heparinlösung gefüllte Spritze eingelegt werden. Die Dosiereinrichtung 20 kann aber auch einen mit Heparinlösung gefüllten Beutel aufweisen.

Das Dialysierflüssigkeitssystem II umfasst eine Einrichtung 17 zum Aufbereiten von Dialysierflüssigkeit mit einer vorgegebenen Temperatur. Die frische Dialysierflüssigkeit strömt über eine Dialysierflüssigkeitszuführleitung 18 in das zweite Kompartiment 4 und die gebrauchte Dialysierflüssigkeit aus dem zweiten Kompartiment 4 der Blutbehandlungseinheit 1 über eine Dialysierflüssigkeitsabführleitung 19 zu der Einrichtung 17 zum Aufbereiten von Dialysierflüssigkeit. Die Einrichtung 17 zum Aufbereiten von Dialysierflüssigkeit ist über eine nicht dargestellte Datenleitung mit der Steuer- und Auswerteeinheit 12 verbunden, so dass die Steuer- und Auswerteeinheit 12 die Temperatur und Zusammensetzung der Dialysierflüssigkeit steuern kann.

Die Messungen für die Bestimmung eines hämodynamischen Parameters der Blutbehandlung können anstelle im extrakorporalen Blutkreislauf I auch im Dialysierflüssigkeitssystem II durchgeführt werden. Diese Verfahren gehören zum Stand der Technik. So kann z.B. durch Messung der Clearance bei normalem und bei umgekehrtem Blutfluss der Fistelfluss bestimmt werden.

Für den Fall einer Hämofiltration braucht eine Einrichtung zum Aufbereiten von Dialysierflüssigkeit und eine zu dem zweiten Kompartiment der Blutbehandlungseinheit führende Dialysierflüssigkeitszuführleitung nicht vorhanden zu sein. Ultrafiltrat kann aus dem zweiten Kompartiment der Blutbehandlungseinheit über die Dialysierflüssigkeitsabführleitung abgezogen werden. Für eine Blutbehandlung, bei der ein diffusiver oder konvektiver Austausch nicht stattfindet, braucht auch ein zweites Kompartiment und eine Dialysierflüssigkeitszu- und abführleitung nicht vorhanden zu sein. Für die Erfindung ist nur entscheidend, dass die Bestimmung des hämodynamischen Parameters eine Flussumkehr erfordert. Für die Erfindung ist nicht entscheidend, ob für die Bestimmung des hämodynamischen Parameters im Blut eine physikalische oder chemische Kenngröße verändert wird. Die Messungen der physikalischen oder chemischen Kenngröße können auf der Blutseite oder Dialysatseite erfolgen.

Bei dem vorliegenden Ausführungsbeispiel fungiert die Einrichtung 17 zum Aufbereiten von Dialysierflüssigkeit auch als Einrichtung zur Veränderung einer physikalischen oder chemischen Eigenschaft des Bluts. Hierfür verändert die Einrichtung 17 zum Aufbereiten von Dialysierflüssigkeit kurzzeitig die Temperatur oder Zusammensetzung der Dialysierflüssigkeit im Dialysierflüssigkeitssystem II, was eine Änderung einer physikalischen oder chemischen Eigenschaft im extrakorporalen Blutkreislauf I zur Folge hat. Die Änderung der physikalischen oder chemischen Eigenschaft im Blut, beispielsweise die Temperatur des Bluts (Temperaturbolus), wird mit der ersten oder zweiten Messeinrichtung 14A, 14B in der ersten und zweiten Blutleitung 5, 7 gemessen. Alternativ kann die Änderung der physikalischen oder chemischen Eigenschaft des Blutes auch durch externe Elemente wie am Blutschlauchsystem angebrachte Peltierelemente oder die Infusion einer physiologischen Lösung über eine in der ersten oder zweiten Blutleitung gelegene Zugabestelle erfolgen.

Die Laufrichtung und Förderrate der Blutpumpe 10 kann mit der Steuer- und Auswerteeinheit 12 geregelt werden. Hierzu können die Druckwerte der Drucksensoren 13A, 13B verwendet werden, um in bekannter Weise aus Umdrehungszahl der Pumpe und Unterdruck auf der jeweiligen Saugseite den effektiven Blutfluss zu bestimmen und zu korrigieren.

Der zeitliche Verlauf der physikalischen oder chemischen Eigenschaft des entnommenen Bluts und des zurückgegebenen Bluts wird mit den am Blutschlauchsystem vorgesehenen Messeinrichtungen 14A, 14B gemessen und an die Steuer- und Auswerteeinheit 12 übermittelt. Bei den Messeinrichtungen 14A, 14B kann es sich beispielsweise um Temperatursensoren, optische oder spektroskopische Sensoren oder Dichtesensoren, beispielsweise Ultraschallsensoren, handeln. Ebenso können Leitfähigkeitsmesszellen verwendet werden, um mit den betreffenden physikalischen oder chemischen Eigenschaften des Bluts korrelierende Größen zu bestimmen. Anstelle der blutseitigen physikalischen oder chemischen Kenngrößen können auch dialysatseitige physikalische oder chemische Kenngrößen gemessen werden, die mit den blutseitigen physikalischen oder chemischen Kenngrößen korrelieren. Hierzu können Messwertaufnehmer zum Messen der Kenngrößen im Dialysierflüssigkeitssystem II, beispielsweise in einer oder beiden Dialysierflüssigkeitsleitungen 18 und 19 vorgesehen sein.

Bei dem vorliegenden Ausführungsbeispiel wird als hämodynamischer Parameter der Fluss im Gefäßzugang (Shuntfluss) bestimmt. Die Bestimmung des Shuntflusses kann mit dem Verfahren erfolgen, das in der DE 195 28 907 A1 beschrieben ist. Die theoretischen Grundlagen des Verfahrens sind in dem Artikel ASAIO journal 1998, Measurement of Access Flow During Hemodialysis Using the Constant Infusion Approach, Daniel Schneditz, Zuoheng Fan, Allen Kaufmann, and Nathan W. Levin, Seiten 74 ff. beschrieben.

Nachfolgend wird ein erstes Ausführungsbeispiel beschrieben. Der Ablauf der Shuntflussmessung wird durch die Steuer- und Auswerteeinheit 12 gesteuert. Zunächst wird die Blutpumpe 10 in der "normalen" Förderrichtung betrieben. In dieser initialen Phase kann die Steuer- und Auswerteeinheit 12 bei laufender Blutpumpe 10 die Dosiereinrichtung 20 aktivieren, so dass dem Blut des Patienten eine gerinnungshemmende Substanz zugegeben wird, die sich auch an die Oberflächen des extrakorporalen Systems, insbesondere an das Lumen der Dialysatorfasern, anlagern kann. Eine derartige initiale Phase ist aber nicht zwingend. Das Antikoagulanz kann auch kontinuierlich zugegeben werden. Die Zugabe von Antikoagulanzien ist für das Verfahren nicht entscheidend. Der Patient kann auch schon vor der Dialyse hinreichend antikoaguliert sein, z.B. bei Verwendung von Macumar.

Nach Ablauf der initialen Phase steuert die Steuer- und Auswerteeinheit 12 die Blutpumpe derart an, dass der Blutfluss umgekehrt wird. Die Temperatur oder Zusammensetzung der Dialysierflüssigkeit wird bei umgekehrtem Blutfluss kurzzeitig verändert. Der Temperaturbolus oder Leitfähigkeitssprung überträgt sich infolge des Wärmeaustausches am Dialysator auf den extrakorporalen Blutkreislauf I. Die Messeinrichtungen 14A, 14B in der ersten und zweiten Blutleitung 5, 7 erfassen die Änderungen der physikalischen oder chemischen Kenngröße in der ersten und zweiten Blutleitung. Die Messwerte werden in einer Speichereinheit 12A der Steuer- und Auswerteeinheit 12 gespeichert. Daraufhin wird die Blutpumpe 10 wieder in der normalen Förderrichtung betrieben. Die Temperatur oder Zusammensetzung der Dialysierflüssigkeit wird bei normalem Blutfluss kurzzeitig verändert und die Temperatur oder Zusammensetzung der Dialysierflüssigkeit in der ersten und zweiten Blutleitung gemessen. Alternativ kann der Verlauf der Leitfähigkeit der Dialysierflüssigkeit mit nicht gezeigten Leitfähigkeitszellen, die in der Dialysierflüssigkeitszuführleitung 18 und der Dialysierflüssigkeitsabführleitung 19 vorgesehen sind, gemessen werden. Die Messwerte werden wieder in der Speichereinheit 12A der Steuer- und Auswerteeinheit 12 gespeichert. Die Steuer- und Auswerteeinheit 12 berechnet nunmehr auf der Grundlage der Messwerte der Messeinrichtungen unter Anwendung einer geeigneten Ablaufsteuerung und entsprechender Auswerteverfahren den Fluss im Gefäßzugang. Das Ergebnis kann auf einer Anzeigeeinheit 21, die über eine Datenleitung 22 mit der Steuer- und Auswerteeinheit 12 verbunden ist, angezeigt oder über eine andere Art der Kommunikation, beispielsweise über ein Netzwerk, ausgegeben werden.

Auf der Anzeigeeinheit 21 können auch eventuelle Fehlermeldungen oder Handlungsanweisungen dem Anwender übermittelt werden. Anstelle einer Anzeigeeinheit kann auch eine Alarmeinheit vorgesehen sein, auf der ein akustischer oder optischer oder taktiler Alarm gegeben wird, beispielsweise bei einer Verstopfung des Filters.

Das Auswertverfahren zur Berechnung des Shuntflusses aus den Messwerten ist in der DE 195 28 907 A1 beschrieben, auf die ausdrücklich Bezug genommen wird.

Die Steuer- und Auswerteeinheit 12 stellt während der Umkehr der Flussrichtung sicher, dass die für Dialysebehandlungen vorgesehenen Schutzeinrichtungen 16A, 16B an die Umkehr der Blutflussrichtung angepasst werden. Hierzu gehört insbesondere die arterielle und venöse Drucküberwachung, deren Bedeutung bei der Flussumkehr vertauscht wird. Während auf der Saugseite der Blutpumpe ("arteriell") ein Unterdruck anliegt, ist der Druck auf der Rückgabeseite ("venös") positiv. Zugleich dient der venöse Druck zur Detektion eventueller Nadeldiskonnektionen. Die Steuer- und Auswerteeinheit 12 ist daher derart konfiguriert, dass das vorgegebene arterielle und venöse Druckgrenzwertfenster bei einer Flussumkehr vertauscht werden. Mit den Algorithmen zur Detektion einer Nadeldiskonnektion, die auf einer dynamischen Signalanalyse beruhen und/oder das absolute Unterschreiten einer unteren Druckschwelle auf der Rückgabeseite überwachen, werden im Normalbetrieb anstelle der Messwerte des venösen Sensors nun die Messwerte des arteriellen Sensors ausgewertet. Zudem erfolgt nach der Flussumkehr eine Überwachung auf Luftinfusion auf der im Normalbetrieb arteriellen Seite.

Bei dem vorliegenden Ausführungsbeispiel erfolgt die Messung zunächst bei einem umgekehrten Blutfluss. Es ist aber auch möglich, dass die Messung zunächst mit einem normalen Blutfluss erfolgt. Der Betrieb der Blutbehandlungsvorrichtung nach der initialen Phase zunächst mit einem umgekehrten Blutfluss hat aber den Vorteil, dass die Umschaltung auf den umgekehrten Blutfluss während der Blutbehandlung so früh wie möglich erfolgt, d. h. nicht erst dann, wenn sich bei einem vorausgehenden normalen Blutfluss schon Gerinnsel haben bilden können.

Die Blutbehandlungsvorrichtung weist eine Eingabeeinheit 23 auf, die über eine Datenleitung 24 mit der Steuer- und Auswerteeinheit 12 verbunden ist. Die Eingabeeinheit 23 kann beispielsweise eine Tastatur 23A und/oder einen berührungsempfindlichen Bildschirm 23B und/oder eine Vorrichtung 23C zum Lesen eines Datenträgers aufweisen. Mit der Eingabeeinheit 23 kann der Anwender ein Zeitintervall eingeben, das der Anwender unter Berücksichtigung verschiedener patientenspezifischer oder systemspezifischer Faktoren festlegen kann. Das eingegebene Zeitintervall wird in dem Speicher 12A der Steuer- und Auswerteeinheit 12 gespeichert.

Die Steuer- und Auswerteeinheit 12 ist derart konfiguriert, dass der Betrieb der Blutpumpe 10 in dem Betriebsmodus mit einem umgekehrten Blutfluss nur innerhalb des mit der Eingabeeinheit 23 eingegebenen Zeitintervalls freigegeben ist. Bei dem vorliegenden Ausführungsbeispiel ist der Anfang des Zeitintervalls der Zeitpunkt des Beginns der initialen Phase der Blutbehandlung.

Die Steuer- und Auswerteeinheit 12 umfasst ein Zeitglied, das mit dem Beginn der initialen Phase der Blutbehandlung in Gang gesetzt wird. Bevor der Blutfluss umgekehrt wird, überprüft die Steuer- und Auswerteeinheit 12, ob das vom Anwender vorgegebene Zeitintervall abgelaufen ist. Wenn dies der Fall ist, verhindert die Steuer- und Auswerteeinheit 12 die Umkehrung des Blutflusses, d. h. nach Ablauf des Zeitgliedes ist eine Bestimmung des hämodynamischen Parameters auf der Grundlage einer Messung mit einem umgekehrten Blutfluss nicht möglich.

Die Steuer- und Auswerteeinheit 12 kann derart konfiguriert sein, dass am Ende des Zeitintervalls, wenn die Blutpumpe 10 in dem Betriebsmodus mit einem umgekehrten Blutfluss bereits betrieben wird, ein Steuersignal erzeugt wird. Dieses Steuersignal kann veranlassen, dass der Betrieb der Blutpumpe unterbrochen wird, wodurch die Messung abgebrochen wird. Das Steuersignal kann auch eine Alarmeinheit 25 aktivieren, die über eine Datenleitung 26 mit der Steuer- und Auswerteeinheit 12 verbunden ist. Der Abbruch der Messung kann auch auf der Anzeigeeinheit 21 angezeigt werden.

Die Steuer- und Auswerteeinheit 12 kann auch derart konfiguriert sein, dass die Sperrung der Umkehr des Blutflusses vom Anwender aufgehoben werden kann, beispielsweise durch eine entsprechende Eingabe mit der Eingabeeinheit, wenn sichergestellt ist, dass sich die Blutgerinnsel, beispielsweise durch Verabreichen eines Antikoagulanz, aufgelöst haben.

Die oben beschriebene Messung nach dem bekannten Verfahren der Thermodilution erfordert eine relativ lange Zeitdauer, die zwischen 8 und 10 Minuten liegen kann. Der hämodynamische Parameter, insbesondere die Rezirkulation, kann aber auch mit den bekannten Verfahren erfolgen, bei denen eine physikalische oder chemische Kenngröße direkt im extrakorporalen Blutkreislauf verändert wird, beispielsweise durch die Infusion einer physiologischen Lösung an einer an der Blutleitung vorgesehenen Zugabestelle. Die Messdauer ist dann relativ kurz.

Zur Durchführung der Messung schaltet die Steuer- und Auswerteeinheit 12 die Blutpumpe 10 zu Beginn der Blutbehandlung oder nach einer initialen Phase in den umgekehrten Blutfluss. Nach der Umkehrung des Blutflusses wird dem Blut in Rückgaberichtung, d.h. in der ersten Blutleitung 8, ein Bolus der physiologischen Lösung zugegeben und die Änderung in der Entnahmerichtung, d.h. in der zweiten Blutleitung gemessen. Da bei der relativ kurzen Messdauer der kardiopulmonare Rezirkulationsanteil zu vernachlässigen ist, kann der Shuntfluss nach der Messung mit dem umgekehrten Blutfluss bereits mit hinreichender Genauigkeit bestimmt werden. Eine Messung bei normalem Blutfluss ist dann nicht mehr erforderlich (Lopet et al, "Vascular acess monitoring evaluated from automated recirculation measurement", EDTNA-ERCA JOURNAL 27, S. 17 ff.).

## Patentansprüche

1. Vorrichtung zur extrakorporalen Blutbehandlung mit
einer Blutbehandlungseinheit (1), die mindestens ein Kompartiment (3) aufweist,
einer an einen Anschluss des Kompartiments (3) angeschlossenen ersten Blutleitung (5), die einen ersten Patientenanschluss (8) aufweist,
einer an einen Anschluss des Kompartiments (3) angeschlossenen zweiten Blutleitung (8), die einen zweiten Patientenanschluss (9) aufweist,
einer Blutpumpe zum Fördern von Blut,
einer Einrichtung (14A, 14B) zum Messen einer physikalischen oder chemischen Kenngröße,
einer mit der Blutpumpe (10) und der Einrichtung zum Messen einer physikalischen oder chemischen Kenngröße verbundenen Steuer- und Auswerteinheit (12), die derart konfiguriert ist, dass
die Blutpumpe (10) für einen Messzyklus von zwei Messzyklen in einem Betriebsmodus mit einem umgekehrten Blutfluss betrieben wird, so dass Blut von dem zweiten Patientenanschluss (9) zu der Blutbehandlungseinheit (1) und von der Blutbehandlungseinheit zu dem ersten Patientenanschluss (8) strömt, und
die Blutpumpe (10) für den anderen Messzyklus der beiden Messzyklen in einem Betriebsmodus mit einem normalen Blutfluss betrieben wird, so dass Blut von dem ersten Patientenanschluss (8) zu der Blutbehandlungseinheit (1) und von der Blutbehandlungseinheit zu dem zweiten Patientenanschluss (9) strömt, wobei aus der gemessenen physikalischen oder chemischen Kenngröße in dem Messzyklus bei normalem und umgekehrtem Blutfluss ein hämodynamischer Parameter bestimmt wird,
oder
die Blutpumpe (10) für einen Messzyklus in einem Betriebsmodus mit einem umgekehrten Blutfluss betrieben wird, so dass Blut von dem zweiten Patientenanschluss (9) zu der Blutbehandlungseinheit (1) und von der Blutbehandlungseinheit zu dem ersten Patientenanschluss (8) strömt, wobei aus der gemessenen physikalischen oder chemischen Kenngröße in dem Messzyklus bei umgekehrtem Blutfluss ein hämodynamischer Parameter bestimmt wird,
wobei die Vorrichtung zur extrakorporalen Blutbehandlung eine Eingabeeinheit (23) zur Eingabe eines Zeitintervalls aufweist,
**dadurch gekennzeichnet, dass**
die Steuer- und Auswerteeinheit (12) derart konfiguriert ist, dass der Betrieb der Blutpumpe (10) in dem Betriebsmodus mit einem umgekehrten Blutfluss nur innerhalb des mit der Eingabeeinheit (23) eingegebenen Zeitintervalls freigegeben ist.

2. Vorrichtung zur extrakorporalen Blutbehandlung nach Anspruch 1, **dadurch gekennzeichnet, dass** die Steuer- und Auswerteeinheit (12) derart konfiguriert ist, dass zu Beginn der Blutbehandlung oder nach Ablauf einer initialen Phase der Blutbehandlung, in der die Blutpumpe (10) mit einem normalen Blutfluss betrieben wird, die Blutpumpe (10) in einem Betriebsmodus mit einem umgekehrten Blutfluss betrieben wird, wobei der hämodynamische Parameter aus der gemessenen physikalischen oder chemischen Kenngröße in dem Messzyklus bei umgekehrtem Blutfluss bestimmt wird.

3. Vorrichtung zur extrakorporalen Blutbehandlung nach Anspruch 1, **dadurch gekennzeichnet, dass** die Steuer- und Auswerteeinheit (12) derart konfiguriert ist, dass zu Beginn der Blutbehandlung oder nach Ablauf einer initialen Phase der Blutbehandlung, in der die Blutpumpe (10) mit einem normalen Blutfluss betrieben wird, die Blutpumpe (10) für einen ersten Messzyklus in einem Betriebsmodus mit einem umgekehrten Blutfluss betrieben wird, und die Blutpumpe (10) für einen zweiten Messzyklus in einem Betriebsmodus mit einem normalen Blutfluss betrieben wird, wobei der hämodynamische Parameter aus der gemessenen physikalischen oder chemischen Kenngröße in dem Messzyklus bei normalem und umgekehrtem Blutfluss bestimmt wird.

4. Vorrichtung zur extrakorporalen Blutbehandlung nach einem der Ansprüche 1 bis 3, **dadurch gekennzeichnet, dass** die Vorrichtung zur extrakorporalen Blutbehandlung eine Dosiereinrichtung (20) für die Zugabe einer gerinnungshemmenden Substanz in das Blut aufweist, wobei die Steuer- und Auswerteeinheit (12) derart konfiguriert ist, dass die Dosiereinrichtung (20) für die Zugabe einer gerinnungshemmenden Substanz in der initialen Phase der Blutbehandlung betrieben wird.

5. Vorrichtung zur extrakorporalen Blutbehandlung nach Anspruch 4, **dadurch gekennzeichnet, dass** die Blutpumpe (10) in der initialen Phase der Blutbehandlung in einem Betriebsmodus mit einem normalen Blutfluss betrieben wird.

6. Vorrichtung zur extrakorporalen Blutbehandlung nach einem der Ansprüche 1 bis 5, **dadurch gekennzeichnet, dass** die Steuer- und Auswerteeinheit (12) derart konfiguriert ist, dass am Ende des Zeitintervalls ein Steuersignal erzeugt wird, wenn die Blutpumpe (10) in dem Betriebsmodus mit einem umgekehrten Blutfluss betrieben wird.

7. Vorrichtung zur extrakorporalen Blutbehandlung nach einem der Ansprüche 1 bis 6, **dadurch gekennzeichnet, dass** die Steuer- und Auswerteeinheit (12) einen Speicher (12A) aufweist, wobei die Steuer- und Auswerteeinheit (12) derart konfiguriert ist, dass die Steuer- und Auswerteeinheit das Zeitintervall aus der Eingabeeinheit (23) in den Speicher einliest.

8. Vorrichtung zur extrakorporalen Blutbehandlung nach einem der Ansprüche 1 bis 7, **dadurch gekennzeichnet, dass** die Eingabeeinheit (23) eine Tastatur und/oder einen berührungsempfindlichen Bildschirm und/oder eine Vorrichtung zum Lesen eines Datenträgers aufweist.

9. Vorrichtung zur extrakorporalen Blutbehandlung nach einem der Ansprüche 1 bis 8, **dadurch gekennzeichnet, dass** die Vorrichtung zur extrakorporalen Blutbehandlung eine Vorrichtung (15) zum Sammeln von Blutgerinnseln in der zweiten Blutleitung (7) aufweist.

10. Vorrichtung nach einem der Ansprüche 1 bis 9, **dadurch gekennzeichnet, dass** der hämodynamische Parameter der Shuntfluss ist.

## Claims

1. Device for extracorporeal blood treatment comprising
a blood treatment unit (1) comprising at least one compartment (3),
a first blood line (5) which is connected to a connection of the compartment (3) and comprises a first patient connection (8),
a second blood line (8) which is connected to a connection of the compartment (3) and comprises a second patient connection (9),
a blood pump for conveying blood,
an apparatus (14A, 14B) for measuring a physical or chemical characteristic variable,
a control and evaluation unit (12) which is connected to the blood pump (10) and to the apparatus for measuring a physical or chemical characteristic variable and is configured such that,
for one measurement cycle of two measurement cycles, the blood pump (10) is operated in an operating mode involving a reversed blood flow, such that blood flows from the second patient connection (9) to the blood treatment unit (1) and from the blood treatment unit to the first patient connection (8), and,
for the other measurement cycle of the two measurement cycles, the blood pump (10) is operated in an operating mode involving a normal blood flow, such that blood flows from the first patient connection (8) to the blood treatment unit (1) and from the blood treatment unit to the second patient connection (9), a haemodynamic parameter being determined from the measured physical or chemical characteristic variable in the measurement cycle when the blood flow is normal and when the blood flow is reversed,
or,
for one measurement cycle, the blood pump (10) is operated in an operating mode involving a reversed blood flow, such that blood flows from the second patient connection (9) to the blood treatment unit (1) and from the blood treatment unit to the first patient connection (8), a haemodynamic parameter being determined from the measured physical or chemical characteristic variable in the measurement cycle when the blood flow is reversed,
the device for extracorporeal blood treatment comprising an input unit (23) for inputting a time interval,
**characterised in that** the control and evaluation unit (12) is configured such that the operation of the blood pump (10) in the operating mode involving a reversed blood flow is only enabled during the time interval input by means of the input unit (23).

2. Device for extracorporeal blood treatment according to claim 1,
**characterised in that** the control and evaluation unit (12) is configured such that, at the start of the blood treatment or after an initial phase of the blood treatment has been carried out in which the blood pump (10) is operated at a normal blood flow, the blood pump (10) is operated in an operating mode involving a reversed blood flow, the haemodynamic parameter being determined from the measured physical or chemical characteristic variable in the measurement cycle when the blood flow is reversed.

3. Device for extracorporeal blood treatment according to claim 1,
**characterised in that** the control and evaluation unit (12) is configured such that, at the start of the blood treatment or after an initial phase of the blood treatment has been carried out in which the blood pump (10) is operated at a normal blood flow, the blood pump (10) is operated, for a first measurement cycle, in an operating mode involving a reversed blood flow, and the blood pump (10) is operated, for a second measurement cycle, in an operating mode involving a normal blood flow, the haemodynamic parameter being determined from the measured physical or chemical characteristic variable in the measurement cycle when the blood flow is normal and when the blood flow is reversed.

4. Device for extracorporeal blood treatment according to any of claims 1 to 3, **characterised in that** the device for extracorporeal blood treatment comprises a metering apparatus (20) for adding an anticoagulant substance to the blood, the control and evaluation unit (12) being configured such that the metering apparatus (20) is operated in the initial phase of the blood treatment in order for an anticoagulant substance to be added.

5. Device for extracorporeal blood treatment according to claim 4,
**characterised in that**, in the initial phase of the blood treatment, the blood pump (10) is operated in an operating mode involving a normal blood flow.

6. Device for extracorporeal blood treatment according to any of claims 1 to 5, **characterised in that** the control and evaluation unit (12) is configured such that a control signal is generated at the end of the time interval if the blood pump (10) is operated in the operating mode involving a reversed blood flow.

7. Device for extracorporeal blood treatment according to any of claims 1 to 6, **characterised in that** the control and evaluation unit (12) comprises a memory (12A), the control and evaluation unit (12) being configured such that the control and evaluation unit reads the time interval from the input unit (23) into the memory.

8. Device for extracorporeal blood treatment according to any of claims 1 to 7, **characterised in that** the input unit (23) comprises a keypad and/or a touchsensitive screen and/or a device for reading a data medium.

9. Device for extracorporeal blood treatment according to any of claims 1 to 8, **characterised in that** the device for extracorporeal blood treatment comprises a device (15) for collecting blood clots in the second blood line (7).

10. Device according to any of claims 1 to 9, **characterised in that** the haemodynamic parameter is shunt flow.

## Revendications

1. Dispositif pour le traitement extracorporel du sang, comprenant
une unité de traitement du sang (1) présentant au moins un compartiment (3),
une première conduite de sang (5) raccordée à un raccord du compartiment (3), laquelle présente un premier raccord patient (8),
une deuxième conduite de sang (8) raccordée à un raccord du compartiment (3), laquelle présente un deuxième raccord patient (9),
une pompe à sang pour refouler le sang,
un organe (14A, 14B) de mesure d'une grandeur caractéristique physique ou chimique,
une unité de commande et d'évaluation (12) reliée à la pompe à sang (10) et à l'organe de mesure d'une grandeur caractéristique physique ou chimique, qui est configurée de telle sorte que
pendant un cycle de mesure parmi deux cycles de mesure, la pompe à sang (10) est exploitée dans un mode de fonctionnement avec un flux sanguin inversé, de sorte que le sang s'écoule du deuxième raccord patient (9) vers l'unité de traitement du sang (1) et de l'unité de traitement du sang vers le premier raccord patient (8), et
pendant l'autre cycle de mesure parmi les deux cycles de mesure, la pompe à sang (10) est exploitée dans un mode de fonctionnement avec un flux sanguin normal, de sorte que le sang s'écoule du premier raccord patient (8) vers l'unité de traitement du sang (1) et de l'unité de traitement du sang vers le deuxième raccord patient (9), un paramètre hémodynamique étant déterminé à partir de la grandeur caractéristique physique ou chimique mesurée pendant le cycle de mesure avec un flux sanguin normal et inversé, ou
pendant un cycle de mesure, la pompe à sang (10) est exploitée dans un mode de fonctionnement avec un flux sanguin inversé, de sorte que le sang s'écoule du deuxième raccord patient (9) vers l'unité de traitement du sang (1) et de l'unité de traitement du sang vers le premier raccord patient (8), un paramètre hémodynamique étant déterminé à partir de la grandeur caractéristique physique ou chimique mesurée pendant le cycle de mesure avec un flux sanguin inversé,
le dispositif de traitement extracorporel du sang comprenant une unité d'entrée (23) pour entrer un intervalle de temps,
**caractérisé en ce que**
l'unité de commande et d'évaluation (12) est configurée de telle sorte que le fonctionnement de la pompe à sang (10) dans le mode de fonctionnement avec un flux sanguin inversé n'est autorisé que dans l'intervalle de temps entré au moyen de l'unité d'entrée (23).

2. Dispositif de traitement extracorporel du sang selon la revendication 1,
**caractérisé en ce que** l'unité de commande et d'évaluation (12) est configurée de telle sorte qu'au début du traitement du sang ou après l'expiration d'une phase initiale du traitement du sang, pendant laquelle la pompe à sang (10) est exploitée avec un flux sanguin normal, la pompe à sang (10) est exploitée dans un mode de fonctionnement avec un flux sanguin inversé, le paramètre hémodynamique étant déterminé à partir de la grandeur caractéristique physique ou chimique mesurée pendant le cycle de mesure avec un flux sanguin inversé.

3. Dispositif de traitement extracorporel du sang selon la revendication 1,
**caractérisé en ce que** l'unité de commande et d'évaluation (12) est configurée de telle sorte qu'au début du traitement du sang ou après l'expiration d'une phase initiale du traitement du sang, pendant laquelle la pompe à sang (10) est exploitée avec un flux sanguin normal, la pompe à sang (10) est exploitée, pendant un premier cycle de mesure, dans un mode de fonctionnement avec un flux sanguin inversé, et la pompe à sang (10) est exploitée, pendant un deuxième cycle de mesure, dans un mode de fonctionnement avec un flux sanguin normal, le paramètre hémodynamique étant déterminé à partir de la grandeur caractéristique physique ou chimique mesurée pendant le cycle de mesure avec un flux sanguin normal et inversé.

4. Dispositif de traitement extracorporel du sang selon l'une des revendications 1 à 3,
**caractérisé en ce que** le dispositif de traitement extracorporel du sang comprend un organe de dosage (20) destiné à ajouter une substance anticoagulante au sang, l'unité de commande et d'évaluation (12) étant configurée de telle sorte que l'organe de dosage (20) destiné à ajouter une substance anticoagulante est exploité pendant la phase initiale du traitement du sang.

5. Dispositif de traitement extracorporel du sang selon la revendication 4,
**caractérisé en ce que** pendant la phase initiale du traitement du sang, la pompe à sang (10) est exploitée dans un mode de fonctionnement avec un flux sanguin normal.

6. Dispositif de traitement extracorporel du sang selon l'une des revendications 1 à 5,
**caractérisé en ce que** l'unité de commande et d'évaluation (12) est configurée de telle sorte qu'un signal de commande est généré à la fin de l'intervalle de temps lorsque la pompe à sang (10) est exploitée dans le mode de fonctionnement avec un flux sanguin inversé.

7. Dispositif de traitement extracorporel du sang selon l'une des revendications 1 à 6,
**caractérisé en ce que** l'unité de commande et d'évaluation (12) comprend une mémoire (12A), l'unité de commande et d'évaluation (12) étant configurée de telle sorte que l'unité de commande et d'évaluation introduit l'intervalle de temps de l'unité d'entrée (23) dans la mémoire.

8. Dispositif de traitement extracorporel du sang selon l'une des revendications 1 à 7,
**caractérisé en ce que** l'unité d'entrée (23) comprend un clavier et/ou un écran tactile et/ou un dispositif destiné à lire un support de données.

9. Dispositif de traitement extracorporel du sang selon l'une des revendications 1 à 8,
**caractérisé en ce que** le dispositif de traitement extracorporel du sang comporte un dispositif (15) de collecte de caillots sanguins dans la deuxième conduite de sang (7).

10. Dispositif selon l'une des revendications 1 à 9,
**caractérisé en ce que** le paramètre hémodynamique est le flux du shunt.
